# EUROPEAN PATENT SPECIFICATION

(11) **EP 2 445 427 B1**
(45) Date of publication and mention of the grant of the patent: **27.03.2013**
(21) Application number: 10728378.0
(22) Date of filing: 16.06.2010
(51) Int. Cl.: A61B 17/70

(54) **VERTEBRAL OSTEOSYNTHESIS EQUIPMENT**
VORRICHTUNG FÜR WIRBELSÄULENOSTEOSYNTHESE
ÉQUIPEMENT D'OSTÉOSYNTHÈSE VERTÉBRAL

(30) Priority: 22.06.2009 FR 0954227
(43) Date of publication of application: 02.05.2012
(73) Proprietor: Medicrea International, 01700 Neyron (FR)
(72) Inventor: JOUVE, Jean Luc, F-13006 Marseille (FR)
(74) Representative: Jeannet, Olivier
(86) International application number: PCT/IB2010/052700
(87) International publication number: WO 2010/150140

(56) References cited:
- FR-A1- 2 767 263
- FR-A1- 2 783 411
- FR-A1- 2 816 196
- FR-A1- 2 856 270
- US-A- 5 562 662

## Description

The present invention relates to vertebral osteosynthesis equipment.

It is well-known how to achieve vertebral osteosynthesis by means of equipment comprising vertebral anchoring members, notably pedicle screws or laminar hooks, at least one rigid bar for supporting the vertebrae, and parts for connecting this(these) supporting bar(s) to these anchoring members, notably as connecting stirrups and clamping nuts, a piece of equipment of this type is notably described in document WO 98/55038.

It is known how to use a piece of equipment further comprising a set of two hooks, so-called «hook-nippers », comprising two antagonistic hooks connected to each other through a linking rod, and including tightenable means with which the distance of these hooks may be adjusted relatively to each other and the hooks may be immobilized at a determined distance from each other. These hooks are intended to be engaged around both opposite edges of the lamina of a same vertebra, or around laminae around two consecutive vertebrae, and to be immobilized in this engagement position by maneuvering said tightenable means.

Document FR 2 816 196 illustrates existing hook-nippers, in which a first hook comprises a threaded pin providing the link of this hook to a supporting bar, and in which the second hook, of smaller size, has the function of ensuring that the engagement of said first hook is maintained around the lamina of a vertebra or of the transverse apophysis.

Such hook-nippers are used at the top of an assembly with supporting bar(s)/anchoring members when it is not possible to place pedicle screws, notably with regard to the morphology or pathology of the vertebra(e) fitted with an implant, or when it is not desirable to place pedicle screws, notably with regard to the poor quality of the bone. Further, the placement of said hooks may be intended, having the advantage of not requiring that the resection and piercing work required by the placement of pedicle screws be carried out on the bone of the vertebrae.

With the aforementioned existing hook-nippers, the rod connecting both hooks is caused to be subject to significant and repeated forces transmitted through vertebra(e) located at the end of the rigid assembly, these vertebrae remaining relatively mobile. These forces are all the more significant since the distance separating the hooks is significant and may lead to the breakage of this rod, which involves surgical re-operation.

The documents FR 2 767 263, FR 2 783 411 and US 5 562 662 disclose various devices according to the prior art.

The aim of the present invention is to find a remedy to the drawback above-mentioned.

The equipment to which it relates, comprises in a way known per se, vertebral anchoring members, notably pedicle screws or hooks, at least one rigid bar for supporting the vertebrae, connecting parts for connecting this or these bars to these anchoring members, and at least one assembly with two antagonistic hooks connected to each other through a linking rod, this assembly comprising tightenable means with which the distance of these hooks relatively to each other can be adjusted and the hooks can be immobilized at a determined distance from each other, a first one of these hooks comprising a member for its connection to a supporting bar.

According to the invention.
- the second hook of said assembly comprises a connecting member for connecting to said supporting bar, and
- this supporting bar has a length such that it extends, after assembly on said vertebral anchoring members, along the connecting members of both hooks of said assembly, and such that it can be connected to these connecting members.

Thus, in the equipment according to the invention, the supporting bar is not only connected to said first hook, but also to said second hook. The result of this is that it is the supporting bar which is subject to the forces exerted by the vertebra(e) and not the linking rod connecting both hooks to each other. Consequently, the risk of breakage of this rod is strongly reduced.

The connecting member which said first hook or said second hook comprises may be integral with this hook or may be formed by a part separated from this hook and mobile relatively to it, notably a jointed part. In this second case, this connecting member may notably be in the form of a threaded pin forming an end portion of spherical shape or as a spherical cap, and the hook may comprise a body having a cavity for receiving this end portion with a joint, said cavity being, after receiving the end portion, closed on the latter, notably by crimping a wall which the hook comprises around said end portion.

Said cavity may notably be laid out by a linking part capable of being screwed into a tapped aperture which the body of the hook comprises.

Preferably, said second hook is formed by a separate part of said linking rod and is connected to the latter through limited pivoting means allowing its limited pivoting relatively to this rod in at least one plane.

This limited pivoting possibility allows this second hook to be oriented relatively to the linking rod, by which it is possible to ensure perfect engagement of the hook around the lamina of a vertebra regardless of the position of the linking rod relatively to the vertebra(e) fitted with an implant.

By "limited pivoting", pivoting according to an angle of the order of 5-20° relatively to the longitudinal axis of the linking rod should be understood.

According to a preferred embodiment of the invention, in this case of limited pivotability of the second hook, said second hook comprises a body pierced right through with a conduit into which said linking rod may be engaged, this conduit comprising a shoulder and the linking rod comprising a widened end portion intended to bear against this shoulder, said conduit as well as said rod, outside this widened end portion, having respective cross-sections allowing a displacement of the rod relatively to the body of the hook in said at least one plane.

This conduit may have a conical portion allowing said second hook to move relatively to the linking rod.

Said widened end portion may be sphere-shaped and said shoulder may then have a span as a mating hollow sphere, for receiving this widened end portion.

These respective spherical shapes ensure a large contact surface area between the body of the hook and the widened end portion formed by the rod and promotes the movement of the rod relatively to the hook.

Advantageously, said two-hook assembly comprises means with which it is possible to ensure the assembling of the linking rod with said second hook so as to avoid any disassembling of both of these parts during the placement of this assembly. In the aforementioned preferred embodiment, these means comprise a tapped portion laid out in said conduit, on the side of this conduit opposite to said first hook, and a threaded plug capable of being screwed into this tapped portion.

The invention will be well-understood and other features and advantages thereof will become apparent, with reference to the schematic appended drawing illustrating as a non-limiting example, a preferred embodiment of the equipment to which it relates.
Fig. 1 is a partly exploded perspective view of a two-hook assembly which this equipment comprises;
Fig. 2 is an exploded side view of this assembly with a partial sectional view of portions of the hooks comprised by this assembly;
Fig. 3 is a view of this assembly similar to Fig. 2, in the assembled condition;
Fig. 4 is a sagittal view of a vertebral column portion on which the intention is to place at least one rigid bar for supporting a series of vertebrae and at least one two-hook assembly as shown in Figs. 1-3; in this figure, a vertebral anchoring member of a supporting bar is in place, as well as a two-hook assembly;
Fig. 5 is a view similar to Fig. 4, after placement of said supporting bar, and
Fig. 6 is a view similar to Fig. 5, after a section of threaded proximal pins comprised by said vertebral anchoring member and both hooks of said two-hook assembly.

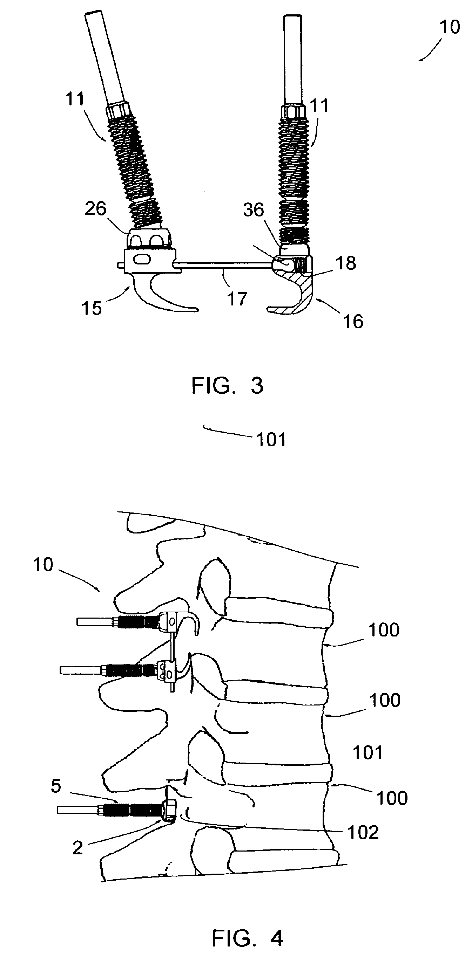

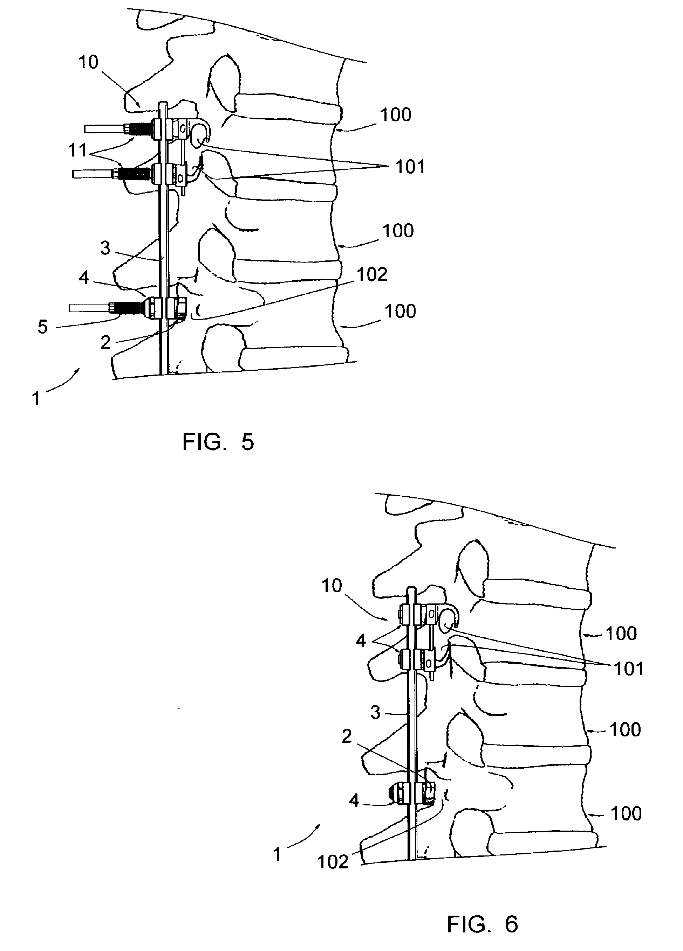

Fig. 5 illustrates osteosynthesis equipment 1 comprising vertebral anchoring members 2 (only one is visible in the figure), at least one rigid bar 3 for supporting vertebrae 100 fitted with an implant, parts 4 for connecting this(these) supporting bar(s) 3 to these anchoring members 2, and at least one two-hook assembly 10. For clarity of the drawing, the transverse apophyses of the vertebrae 100 have not been illustrated in Figs. 4-6, and the laminae 101 of the vertebrae 100 on which the hooks of the assembly 10 are engaged, have been represented very schematically.

The vertebral anchoring members 2 are notably in the form of pedicle screws, i.e. screws intended to be placed in the pedicles 102 of the vertebrae 100. Such a screw includes a threaded proximal pin 5 allowing the assembly of said connecting parts 4. These screws may notably be of the so-called "polyaxial" type, i.e. in which the threaded proximal pins 5 are jointed relatively to the bodies of the screws, as this is described in document WO 98/55038.

Said connecting parts 4 are notably in the form of connecting stirrups and of clamping nuts as also described by this same prior document.

It appears in Figs. 5 and 6 that, specifically to the present invention, each hook of the assembly 10 comprises a proximal pin 11 for connection to the supporting bar 3, and that this supporting bar 3 has a length such that it extends, after assembly on said vertebral anchoring members 2, facing these proximal pins 11 and that it may be connected to the latter by means of connecting stirrups and of clamping nuts similar to those allowing the bar 3 to be connected to the members 2.

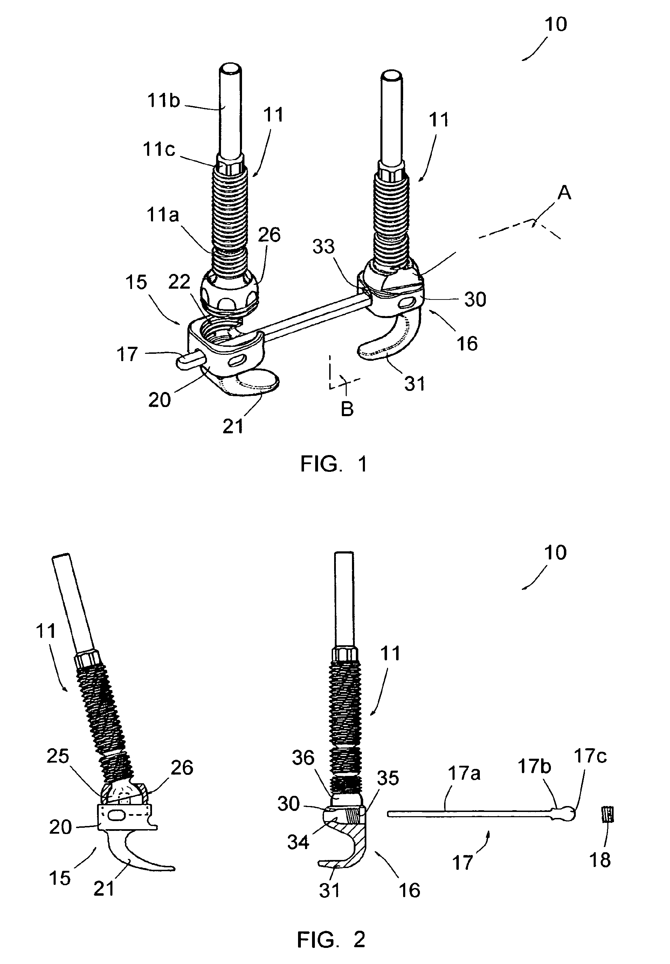

Figs. 1-3 more particularly show an assembly 10.

Fig. 2 shows that this assembly 10 comprises, in addition to the aforementioned pins 11, a first hook 15, a second hook 16, a rod 17 independent of the hooks 15, 16, intended to connect both of these hooks 15, 16 to each other, and a threaded plug 18.

The hook 15 comprises a body 20 and a curved lower portion 21 forming the hook strictly speaking.

The body 20 is intended to be crossed by the rod 17 with the possibility of a limited pivoting displacement of the hook 15 relatively to this rod. For this purpose it comprises a conduit passing right through it, as a circular sector, this conduit having on the side of the body 20 intended to be opposite to the hook 16, a cross-section relatively adjusted to the cross-section of the rod 17, and running while widening in the direction of the side of the body 20 intended to be turned towards the hook 16, in order to open out on the outside through an oblong aperture.

Moreover the body 20 has a tapped circular cavity 22 opening out in its face opposite to the one including the curved portion 21 which joins up with the aforementioned conduit.

The proximal pin 11 of the hook 15 is of the polyaxial type, i.e. it is connected to the body 20 with a possibility of a joint. As this is visible in Fig. 2, it has at one end a joint portion 25 substantially hemisphere-shaped, the terminal face of which has a central substantially square housing, this portion being received into a cavity which is interiorly delimited by a connecting part 26. This linking part 26 comprises a flat bottom including, in said cavity, a central square pad intended to be engaged into the central housing of the joint portion 25. This linking part 26 also comprises a peripheral wall intended, after engagement of the joint portion 25, into said cavity, to be crimped around this portion 25, this crimping ensuring that the latter is retained in the cavity. The respective dimensions of said central square pad and of said central housing are such that the engagement of the pad into the housing does not interfere with the jointing of the pin 11 while achieving blocking of this pin 11 in rotation relatively to the hook 15.

The linking part 26 exteriorly has a threading allowing it to be screwed into the cavity 22 and planar facets allowing it to be taken by a screwing instrument. Complete screwing of the part 26 into the aperture 22 causes the bottom of this part 26 to bear against the rod 17 engaged through the body 20 of the hook 15 and therefore achieves immobilization of this rod relatively to this body. The rod 17 and/or said bottom may have surface asperities or knurling promoting perfect immobilization of the rod 17 relatively to the part 26 and therefore relatively to the body 20.

The proximal pin 11 moreover has, as this is known, a portion 11a of smaller section allowing it to be broken after placing the stirrup and screwing the clamping nut, a smooth proximal portion 11 b allowing the connection of an extension rod facilitating the engagement of said stirrup and of said nut, and an engagement portion with facets 11c.

The hook 16 also comprises a body 30 and a curved lower portion 31 forming the hook strictly speaking.

The body 30 is intended to be crossed by the rod 17 with the possibility of limited pivoting displacement of the hook 16 relatively to this rod. For this purpose it comprises a conduit passing right through it. On the side of the body 30 intended to be turned towards the hook 15, this conduit opens out on the outside through an oblong aperture 33 with a length greater than the cross-section of the rod 17. Interiorly, this conduit has on the side intended to be turned towards the hook 15, a shoulder 34 having a span in the form of a hollow sphere and on the side intended to be opposite to the hook 15, a tapped portion 35.

The proximal pin 11 of the hook 16 is, in the illustrated example, fixed relatively to the body 30 of this hook, and is therefore without any portion of a joint 25. It is connected to the body 30 through a linking portion 36. As for the remainder, it has a structure identical with or very similar to that of the pin 11 of the hook 15.

The rod 17 has a main portion 17a with a substantially rectangular cross-section, and a widened end portion forming a spherical portion 17b and a cylindrical portion 17c. The spherical portion 17b is dimensioned so that during the engagement of the rod 17 into the conduit of the hook 16, it is received against the shoulder 34 laid out in this conduit and it retains the rod 17 axially in this conduit while allowing a displacement of the rod 17 relatively to the hook 16 in a plane A containing the longitudinal axis of the rood 17 and/or in a plane B in which extends the curved portion 31 of the hook 16. The cylindrical portion 17c is intended to extend, when the spherical portion 17b is in contact with the shoulder 34, at the aperture 33 of the body 30.

The threaded plug 18 is intended to be screwed into the tapped portion 35 of the conduit of the hook 16 so as to ensure assembling of the rod 17 engaged into this conduit and of the hook 16, as shown by Fig. 3.

In practice, the vertebral anchoring members 2 are placed on the vertebrae 100 which should be rigidly immobilized by the bar(s) 3 and then one or more assemblies 10 are placed by engaging the hooks 15 and 16 on the lamina 101 of the vertebra 100 located at the end of the vertebral segment which has to be immobilized (cf. Fig. 4). The aforementioned limiting pivotabilities of the hooks 15 and 16 relatively to the rod 17 facilitate the placement of these hooks on the lamina 101.

The bar(s) 3 are then placed on the members 2 by means of stirrups and nuts forming said connecting parts 4; as already mentioned and as shown by Fig. 5, at least one of these bars 3 has a length such that it extends after being assembled on the members 2, facing the pins 11 of both hooks 15 and 16; these pins 11 may therefore also receive stirrups and nuts allowing these hooks 15, 16 to be connected to the bar 3. The result of this is that the bar 3 is the one which is subject to the forces exerted by the vertebra 100 located at the end of the immobilized vertebral segment and not the rod 17.

The assembly is then finalized by breaking the thinned portions of the pins 5 and 11 jutting out beyond the clamping nuts and the stirrups, as shown by Fig. 6.

It appears from the foregoing that the invention provides vertebral osteosynthesis equipment having the determining advantages of strongly reducing the risk of breakage of the linking rod 17 and of facilitating the placement of an assembly 10 on the vertebra lamina 101 or on the laminae 101 of two consecutive vertebrae.

The invention was described above with reference to an embodiment given as a pure example. It is obvious that it is not limited to this embodiment and that it extends to all the other embodiments covered by the appended claims.

## Claims

1. Vertebral osteosynthesis equipment (1), comprising vertebral anchoring members (2), notably pedicle screws or hooks, at least one rigid bar (3) for supporting the vertebrae, connecting parts (4) for connecting this or these bars (3) to these anchoring members (2), and at least one assembly (10) with two antagonistic hooks (15, 16) connected to each other through a linking rod (17), this assembly (10) comprising tightenable means (26) with which the distance of these hooks (15, 16) relatively to each other can be adjusted and the hooks can be immobilized at a determined distance from each other, a first one of these hooks (15, 16) comprising a member (11) for its connection to a supporting bar (3);
**characterized in that**:
- the second hook (15, 16) of said assembly (10) comprises a connecting member (11) for connecting to said supporting bar (3), and
- this supporting bar (3) has a length such that it extends, after assembly on said vertebral anchoring members (2), along the connecting members (11) of both hooks (15, 16) of said assembly (10), and such that it can be connected to these connecting members (11).

2. Equipment (1) according to claim 1, **characterized in that** the connecting member (11) which said first hook (15) or said second hook (16) comprises is integral with this hook.

3. Equipment (1) according to claim 1, **characterized in that** the connecting member which said first hook (15) or said second hook comprises is formed by a part separated from this hook and mobile relatively to it, notably a jointed part.

4. Equipment (1) according to claim 3, **characterized in that** the connecting member which said hook (15) comprises is in the form of a threaded pin (11) forming an end portion (25) of spherical shape or as a spherical cap, and **in that** the hook (15) comprises a body (20) having a cavity for receiving this end portion (25) with a joint, said cavity being, after receiving the end portion (25), closed on the latter, notably by crimping a wall which the hook (15) comprises around said end portion (25).

5. Equipment (1) according to claim 4, **characterized in that** said cavity is laid out by a linking part (26) capable of being screwed into a tapped aperture (22) which the body (20) of the hook (15) comprises.

6. Equipment (1) according to any of claims 1 to 5, **characterized in that** said second hook (16) is formed by a separate part of said linking rod (17) and is connected to the latter through limited pivoting means (33, 34, 17b) allowing its limited pivoting relatively to this rod in at least one plane (A).

7. Equipment (1) according to claim 6, **characterized in that** said second hook (16) comprises a body (30) pierced right through with a conduit into which said linking rod (17) may be engaged, this conduit comprising a shoulder (34) and the linking rod (17) comprising a widened end portion (17b) intended to bear against this shoulder (34), said conduit as well as said rod (17), outside this widened end portion (17b), having respective cross-sections allowing a displacement of the rod (17) relatively to the body (20) of the hook (16) in said at least one plane (A).

8. Equipment (1) according to claim 7, **characterized in that** said widened end portion (17b) is sphere-shaped and **in that** said shoulder (34) has a span as a mating hollow sphere, for receiving this widened end portion (17b).

9. Equipment (1) according to any of claims 6 to 8, **characterized in that** said two-hook assembly (10) comprises means (35, 18) with which it is possible to ensure the assembling of the linking rod (17) with said second hook (16).

10. Equipment (1) according to claim 7 or claim 9, **characterized in that** these means with which it is possible to ensure the assembling of the linking rod (17) with said second hook (16) comprise a tapped portion (35) laid out in said conduit of said second hook (16), on the side of this conduit opposite to said first hook (15), and a threaded plug (18) capable of being screwed into this tapped portion (35).

## Patentansprüche

1. Knochenvereinigung der Wirbel Ausrüstung (1), umfassend Vertebralen Verankerungselemente (2), insbesondere Pedikelschrauben oder Haken, wenigstens eine starre Stange (3) zum Tragen der Wirbel, Verbindungsteilen (4) zum Verbinden dieser oder diese Stangen (3) zu diesen Verankerungsorganen (2), und mindestens eine Anordnung (10) mit zwei antagonistische Haken (15, 16) miteinander verbunden sind durch eine Verbindungsstange (17), wobei diese Anordnung (10) mit festziehbar Mittel (26), mit denen der Abstand dieser Haken (15, 16) relativ zueinander eingestellt werden kann und die Haken in einem bestimmten Abstand voneinander immobilisiert werden können, wobei ein erster dieser Haken (15, 16) mit einem Element (11) für ihre Verbindung mit einer Tragstange (3) umfasst;
**dadurch gekennzeichnet, dass**:
- Der zweite Haken (15, 16) der Anordnung (10) ein Verbindungselement (11) zum Verbinden mit dem Stützstab (3) umfasst, und
- Diese Tragstange (3) eine solche Länge hat, daß es sich nach dem Zusammenbau auf dem Wirbelkörper Verankerungselemente (2), entlang der Verbindungselemente (11) der beiden Haken (15, 16) der Anordnung (10), und derartige dass sie zu diesen Verbindungsteilen (11) verbunden werden.

2. Ausrüstung (1) nach Anspruch 1, **dadurch gekennzeichnet, daß** das Verbindungsglied (11), der erste Haken (15) oder dem zweiten Haken (16) umfasst, wobei die einstückig mit diesem Haken aufweist.

3. Ausrüstung (1) nach Anspruch 1, **dadurch gekennzeichnet, daß** das Verbindungsglied die ersten Haken (15) oder des zweiten Hakens aufweist, indem ein Teil von diesem mobilen Haken und relativ dazu getrennt sind, insbesondere zu einem zusammengesetzten Teils gebildet ist.

4. Ausrüstung (1) nach Anspruch 3, **dadurch gekennzeichnet, daß** das Verbindungselement, die Haken (15) umfasst, wobei die in Form von einem Gewindestift (11) einen Endabschnitt (25) der Kugelform oder als Kugelkalotte ist **dadurch gekennzeichnet**, , und daß der Haken (15) einen Körper (20) mit einem Hohlraum zur Aufnahme dieses Endabschnitts (25) mit einem Gelenk, wobei der Hohlraum, umfassend nach Aufnahme des Endabschnitts (25), auf letzterem verschlossen, insbesondere durch Crimpen einer Wand, die der Haken (15), die um den Endabschnitt (25) umfasst.

5. Ausrüstung (1) nach Anspruch 4, gekennzeichnet, dass die Kavität erfolgt durch ein Verbindungsteil (26), in einer Gewindebohrung Öffnung (22) geschraubt gelegt geeignet ist, die der Körper (20) des Hakens (15) umfasst.

6. Ausrüstung (1) nach einem der Ansprüche 1 bis 5, **dadurch gekennzeichnet, daß** der zweite Haken (16) durch ein separates Teil der Verbindungsstange (17) gebildet wird und mit dem letzteren durch begrenzte Schwenkmittel verbunden ist (33 gekennzeichnet, 34, 17b) ermöglichen die begrenzte Verschwenkung relativ zu dieser Stange in mindestens einer Ebene (A).

7. Ausrüstung (1) nach Anspruch 6, daß der zweite Haken (16) einen durchbohrten Körper (30) umfasst, mit einer Leitung, in die Verbindungsstange (17) eingreifen kann, werden diese Leitung eine Schulter (34) und die Verbindungsstange (17) mit einem verbreiterten Endabschnitt (17b), gegen diese Schulter (34) tragen bestimmt ist, wobei die Leitung als auch die Stange (17), außerhalb dieses verbreiterte Ende (17b) mit jeweiligen Quer -Abschnitte ermöglicht eine Verschiebung der Stange (17) relativ zum Körper (20) des Hakens (16) in der mindestens einen Ebene (A).

8. Ausrüstung (1) nach Anspruch 7, **dadurch gekennzeichnet, dass** das verbreiterte Ende (17b) kugelförmig ist und daß die Schulter (34) eine Spannweite als Gegenstück Hohlkugel, für den Empfang dieses verbreiterte Ende (17b) hat.

9. Ausrüstung (1) nach einem der Ansprüche 6 bis 8, **dadurch gekennzeichnet, daß** gekennzeichnete Zwei-Haken-Baugruppe (10) Mittel (35, 18) umfaßt mit dem es möglich ist, die Montage der Verbindungsstange (17) mit der gekennzeichnete zweite Haken (16) sicherzustellen.

10. Ausrüstung (1) nach Anspruch 7 oder Anspruch 9, **dadurch gekennzeichnet, daß** diese Mittel mit der es möglich ist, dass die Montage der Verbindungsstange (17) mit dem zweiten Haken (16) zu gewährleisten eine Gewinde (35) vorgesehenen in der Leitung des zweiten Hakens (16), auf der Seite dieser Leitung gegenüber der ersten Haken (15), und ein Gewindestopfen (18) geeignet ist, in diesem abgetrennten Teiles (35) verschraubt ist.

## Revendications

1. Matériel d'ostéosynthèse vertébrale (1), comprenant des éléments (2) d'ancrage vertébral, notamment des vis pédiculaires ou des crochets, au moins une barre rigide (3) d'étayage des vertèbres, des pièces (4) de connexion de cette ou ces barres (3) à ces éléments d'ancrage (2), et au moins un ensemble (10) à deux crochets (15, 16) antagonistes reliés l'un à l'autre par une tige de liaison (17), cet ensemble (10) comprenant des moyens serrables (26) qui permettent de régler la distance de ces crochets (15, 16) l'un par rapport à l'autre et d'immobiliser les crochets à une distance déterminée l'un de l'autre, un premier de ces crochets (15, 16) comprenant un organe (11) pour sa connexion à une barre d'étayage (3) ;
**caractérisé en ce que** :
- le deuxième crochet (15, 16) dudit ensemble (10) comprend un organe (11) de connexion à ladite barre d'étayage (3), et
- cette barre d'étayage (3) présente une longueur telle qu'elle s'étend, après montage sur lesdits éléments (2) d'ancrage vertébral, en regard des organes de connexion (11) des deux crochets (15, 16) dudit ensemble (10) et qu'elle peut être reliée à ces organes de connexion (11).

2. Matériel (1) selon la revendication 1, **caractérisé en ce que** l'organe de connexion (11) que comprend ledit premier crochet (15) ou ledit deuxième crochet (16) forme corps avec ce crochet.

3. Matériel (1) selon la revendication 1, **caractérisé en ce que** l'organe de connexion que comprend ledit premier crochet (15) ou ledit deuxième crochet est formé par une pièce séparée de ce crochet et mobile par rapport à ce dernier, notamment articulée.

4. Matériel (1) selon la revendication 3, **caractérisé en ce que** l'organe de connexion que comprend ledit crochet (15) est sous la forme d'un pion fileté (11) formant une portion d'extrémité (25) de forme sphérique ou en calotte sphérique, et **en ce que** ce crochet (15) comprend un corps (20) présentant une cavité de réception de cette portion d'extrémité (25) avec articulation, ladite cavité étant, après réception de cette portion d'extrémité (25), refermée sur cette dernière, notamment par sertissage d'une paroi que comprend le crochet (15) autour de ladite portion d'extrémité (25).

5. Matériel (1) selon la revendication 4, **caractérisé en ce que** ladite cavité est aménagée par une pièce de liaison (26) apte à être vissée dans une ouverture taraudée (22) que comprend le corps (20) du crochet (15).

6. Matériel (1) selon l'une des revendications 1 à 5, **caractérisé en ce que** ledit deuxième crochet (16) est formé par une pièce séparée de ladite tige de liaison (17) et est relié à cette dernière par des moyens (33, 34, 17b) de pivotement limité permettant son pivotement limité par rapport à cette tige dans au moins un plan (A).

7. Matériel (1) selon la revendication 6, **caractérisé en ce que** ledit deuxième crochet (16) comprend un corps (30) percé de part en part d'un conduit dans lequel peut être engagée ladite tige de liaison (17), ce conduit comprenant un épaulement (34) et la tige de liaison (17) comprenant une portion d'extrémité élargie (17b) destinée à venir porter contre cet épaulement (34), ledit conduit ainsi que ladite tige (17) en dehors de cette portion d'extrémité élargie (17b) présentant des sections transversales respectives permettant un débattement de la tige (17) par rapport au corps (20) du crochet (16) dans ledit au moins un plan (A).

8. Matériel (1) selon la revendication 7, **caractérisé en ce que** ladite portion d'extrémité élargie (17b) est en forme de sphère et **en ce que** ledit épaulement (34) présente une portée en forme de sphère creuse correspondante, de réception de cette portion d'extrémité élargie (17b).

9. Matériel (1) selon l'une des revendications 6 à 8, **caractérisé en ce que** ledit ensemble (10) à deux crochets (15, 16) comprend des moyens (35, 18) permettant d'assurer l'assemblage de la tige de liaison (17) avec ledit deuxième crochet (16).

10. Matériel (1) selon la revendication 7 et la revendication 9, **caractérisé en ce que** ces moyens permettant d'assurer l'assemblage de la tige de liaison (17) avec ledit deuxième crochet (16) comprennent une portion taraudée (35) aménagée dans ledit conduit du deuxième crochet (16), du côté de ce conduit opposé audit premier crochet (15), et un bouchon fileté (18) propre à être vissé dans cette portion taraudée (35).
